# EUROPÄISCHE PATENTSCHRIFT

(11) **EP 0 567 810 B1**
(45) Veröffentlichungstag und Bekanntmachung des Hinweises auf die Patenterteilung: **17.07.1996**
(21) Anmeldenummer: 93105645.1
(22) Anmeldetag: 06.04.1993
(51) Int. Cl.: C10L 1/22, C10M 133/56, C10L 10/00

(54) **Kraftstoffe und Schmierstoffe, enthaltende N-Alkyl-Carbonsäureamide**
Fuels and lubricants, containing N-alkyl carboxylic acid amide
Carburants et lubrifiants contenant une N-alkyl amide d'acide carboxylique

(30) Priorität: 25.04.1992 DE 4213677
(43) Veröffentlichungstag der Anmeldung: 03.11.1993
(73) Patentinhaber: BASF Aktiengesellschaft, D-67063 Ludwigshafen (DE)
(72) Erfinder: Mohr, Juergen, Dr., W-6718 Gruenstadt (DE); Oppenlaender, Knut, Dr., W-6700 Ludwigshafen (DE); Pander, Hans Joachim, W-6701 Roedersheim-Gronau (DE); Schneider, Rolf, Dr., W-6800 Mannheim 1 (DE); Thomas, Juergen, W-6701 Fussgoenheim (DE); Schreyer, Peter, Dr., W-6940 Weinheim (DE)

(56) Entgegenhaltungen:
- EP-A- 0 355 895
- EP-A- 0 476 485
- DE-A- 2 061 057
- DE-A- 3 611 230
- US-A- 3 996 024
- US-A- 4 743 389

## Beschreibung

Die Erfindung betrifft Kraftstoffe für Verbrennungsmotoren und Schmierstoffe, enthaltend geringe Mengen an N-Alkyl-Carbonsäureamiden.

Vergaser und Einlaßsystem von Ottomotoren, aber auch Einspritzsysteme für die Kraftstoffdosierung in Otto- und Dieselmotoren werden in zunehmendem Maße durch Verunreinigungen belastet, die durch Staubteilchen aus der Luft, unverbrannte Kohlenwasserstoffreste aus dem Brennraum und die in den Vergaser geleiteten Kurbelwellengehäuseentlüftungsase verursacht werden.

Die Rückstände verschieben das Luft-Kraftstoffverhältnis im Leerlauf und im unteren Teillastbereich, so daß das Gemisch fetter, die Verbrennung unvollständiger und wiederum die Anteile unverbrannter oder teilverbrannnter Kohlenwasserstoffe im Abgas größer werden und der Benzinverbrauch steigt.

Es ist bekannt, daß zur Vermeidung dieser Nachteile Kraftstoffadditive zur Reinhaltung von Ventilen und Vergaser bzw. Einspritzsystemen verwendet werden (vgl. z.B.: M. Rossenbeck in Katalysatoren, Tenside, Mineralöladditive, Hrsg. J. Falbe, U. Hasserodt, S. 223 f., G. Thieme Verlag, Stuttgart 1978).

Je nach Wirkungsweise aber auch nach dem bevorzugten Wirkort solcher Detergent-Additive unterscheidet man heute zwei Generationen.

Die erste Additiv-Generation konnte nur die Bildung von Ablagerungen im Ansaugsystem verhindern, nicht aber bereits vorhandene Ablagerungen wieder entfernen, wohingegen die Additive der zweiten Generation beides bewirken können ("keepclean-" und "clean-up-Effekt"), und zwar aufgrund ihrer hervorragenden Thermostabilität, insbesondere auch an Zonen höherer Temperaturen, nämlich an den Einlaßventilen.

Das molekulare Bauprinzip von Kraftstoff-Detergenzien kann verallgemeinernd angegeben werden als Verknüpfung polarer Strukturen mit meist höhermolekularen, unpolaren oder lipohilen Resten.

Vertreter der zweiten Additiv-Generation sind oft Produkte auf der Basis von Polyisobutenen im unpolaren Molekülteil. Hier wiederum sind Additive vom Polyisobutylamin-Typ besonders hervorzuheben. Polyisobutylamine erhält man, ausgehend von Polyisobutenen, im wesentlichen nach zwei Verfahren. Das erste verläuft über eine Chlorierung des polymeren Grundkörpers und anschließenden nukleophilen Ersatz durch Amine oder vorzugsweise Ammoniak. Der Nachteil dieses Verfahrens ist die Verwendung von Chlor und das Auftreten von chlor- oder chloridhaltigen Produkten, die keinesfalls mehr erwünscht sind und soweit irgend möglich gemieden werden (DE-OS 21 29 461, DE-OS 22 45 918).

Im zweiten Verfahren wird ein reaktives Polyisobuten zunächst in einer Oxosynthese carbonyliert und danach in Anwesenheit von Ammoniak aminierend hydriert (DE-OS 36 11 230).

Aus der DE-OS 20 61 057 ist ein Verfahren zur Herstellung von primären Aminen sowie von entsprechenden Formamid-Derivaten und Formiminoestern bekannt, bei dem u.a. auch Polyisobutylen nach der sogenannten "Ritter-Reaktion" zu Aminen umgesetzt wird.

Die "Ritter-Reaktion" ist die Umsetzung von Olefinen mit HCN oder Nitrilen unter saurer Katalyse zu substituierten Amiden, die zu Aminen hydrolysiert werden können.

Diese Reaktion ist beispielsweise beschrieben in Org. React. 17, 213-325 (1969) oder in Houben-Weyl E5, S. 1032-1041 (1985) bzw. Houben-Weyl, XI/1, S. 994 f. (1957).

Die nach der DE-OS 20 61 057 hergestellten Polyisobutylamine entsprechen den Verbindungen nach der DE-OS 22 45 918. Ihre Verwendung als Detergenzien in Kraft- und Schmierstoffen wird auch in der DE-OS 20 61 057 erwähnt. Gemäß dieser Druckschrift ist es aber erforderlich, die zunächst durch die Ritter-Reaktion erhaltenen Formamide zu hydrolysieren. Damit gelangt man, wie auch gemäß der DE-OS 22 45 918 und DE-OS 36 11 230, nur in zwei Stufen vom Olefin zu einem wirksamen Endprodukt.

Darüber hinaus kann die nach der "Ritter-Reaktion" zur Hydrolyse erforderliche Säure nicht in die Reaktion zurückgeführt werden, da sie mit der bei der Hydrolyse entstehenden Ameisensäure verunreinigt ist. Damit entsteht bei dieser Hydrolyse eine in hohem Maße unerwünschte Säure- bzw. Salzfracht.

Der vorliegenden Erfindung lag daher die Aufgabe zugrunde, hochwirksame Detergenzien für Kraft- und Schmierstoffe zur Verfügung zu stellen, die verfahrenstechnisch auf möglichst einfache Weise, d.h. in einem Schritt, aus den entsprechenden Olefinen hergestellt werden können, ohne daß größere Mengen an nicht mehr verwertbarer Salzfracht anfallen.

Diese Aufgabe wird gelöst durch Kraftstoffe oder Schmierstoffe, enthaltend geringe Mengen an Verbindungen der Formeln Ia und/oder Ib 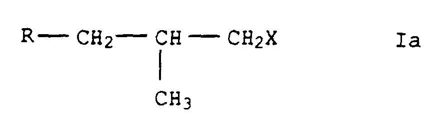 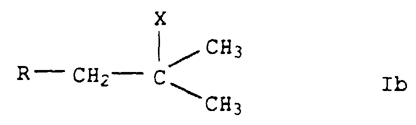 in der
- R: einen aliphatischen, Alkyl-Seitenketten aufweisenden Kohlenwasserstoffrest mit einem Molekulargewicht (Zahlenmittel) von 250 bis 5000 und
- X: die Gruppe 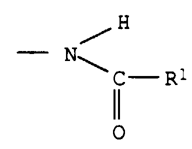 bedeutet, wobei der Rest R¹ Wasserstoff, C₁-C₆-Alkyl, Phenyl oder Alkyl-Phenyl mit 7 bis 14 C-Atomen bedeutet.

Überraschenderweise zeigen bereits die durch die "Ritter-Reaktion" unmittelbar hergestellten N-Alkyl-Carbonsäureamide eine gute Wirkung als Detergenzien.

Als Olefinkomponente für die "Ritter-Reaktion" verwendet man vorzugsweise ein von Isobuten und 0 bis 30 Gew.-% n-Buten abgeleitetes Polyisobuten mit einem mittleren Molekulargewicht zwischen 250 und 5000. Aufgrund des bekannten Mechanismus der "Ritter-Reaktion" ist es dabei nicht unbedingt notwendig, daß das Polyisobuten ein α-Olefin ist.

Als Nitrilkomponente wird bevorzugt Blausäure verwendet, d.h. man erhält in der Umsetzung, die am günstigten schwefelsäurekatalysiert geführt wird, die jeweiligen N-Polyisobutylformamide. Aber auch Nitrile, wie z.B. Acetonitril oder Benzonitril, das alkylsubstituiert sein kann, wobei die Summe der C-Atome der Alkylsubstituenten 1 bis 8 sein kann, sind einsetzbar.

Die N-Alkyl-Carbonsäureamide werden den Kraft- und Schmierstoffen in als Dergens wirksamen Mengen zugesetzt, insbesondere werden sie den Kraftstoffen in Mengen von 50 bis 5000, vorzugsweise 100 bis 2000 ppm und Schmierölen in Mengen von 0,5 bis 10, vorzugsweise 1 bis 5 Gew.-%, bezogen auf das Schmieröl, zugesetzt.

Die in der "Ritter-Reaktion" zur Herstellung der erfindungsgemäßen Additive eingesetzten Olefine (Polyalkylene) werden durch Polymerisation von geradkettigen oder verzweigten monomeren C₂-C₃₀-, bevorzugten C₂-C₆-, insbesondere C₂-C₄-Olefinen hergestellt, wobei die Polymerisation so geführt wird, daß der Kettenabbruch zu einer Doppelbindung führt (z.B. durch kationische oder koordinative Polymerisation).

Als monomere Olefine zur Herstellung der Ausgangskomponente für die "Ritter-Reaktion" werden bevorzugt 1-Alkene eingesetzt, insbesondere Propylen, 1-Buten, Isobuten oder Gemische dieser Olefine.

Ethylen wird nur in Verbindung mit Comonomeren eingesetzt, da reines Polyethylen zu Verbindungen führt, die unter normalen Bedingungen nicht kraftstofflöslich sind.

Die resultierenden Polyalkylene können Homopolymere oder Copolymere sein, die jeweils Alkylseitenketten mit 1 bis 28, bevorzugt 1 bis 4, insbesondere 1 bis 2 C-Atomen aufweisen.

Die als Ausgangskomponente für die "Ritter-Reaktion" bevorzugt eingesetzten Polyisobutene haben ein mittleres Molekulargewicht von 500 bis 5000, insbesondere 800 bis 2000. Sie werden nach bekanntem Verfahren durch kationische Polymerisation von Isobuten erhalten, wobei nach Abbruch der Polymerkette im zuletzt eingebauten Monomeren eine Doppelbindung verbleibt (vgl. z.B.: DE-OS 27 02 604 und EP-A 0 145 235).

Die neuen Additive können auch in Kombination mit anderen bekannten Detergenzien und Dispergatoren in Kraftstoffadditiv-Formulierungen eingesetzt werden.

Insbesondere eignen sich hierzu die an sich an sich bekannten Polyisobutylamine der Formel 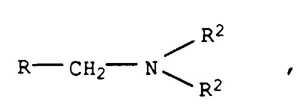 in der R ein von Isobuten und 0 bis 30 Gew.-% n-Buten abgeleiteter Polybutyl- oder Polyisobutylrest und R² Wasserstoff, C₁-C₁₀-Alkyl oder C₁-C₈-Aminoalkyl, das noch durch weitere, Aminogruppen tragende C₁-C₆-Alkylreste substituiert sein kann, bedeutet, um einen sehr guten Detergenseffekt zu erzielen.

Die Prüfung der Produkte als Kraftstoffadditive, besonders auf ihre Eignung als Ventil- und Vergaserreiniger, geschieht mit Hilfe von Motortests, die in Prüfstandversuchen min einem nem 1,2 l Opel-Kadett-Motor gemäß CEC-F-02-T-79 durchgeführt werden.

### Beispiele

### 1. Herstellung eines Polyisobutylformamids

In einem Rührkolben wird eine Mischung aus 200 g konz. Schwefelsäure, 10 g Wasser und 32,4 g wasserfreier Blausäure vorgelegt.

Zu dieser Mischung dosiert man innerhalb von 1,5 Stunden eine Lösung von 400 g Polyisobuten (mittleres Molekulargewicht 950, Jod-Zahl 27,2) in 400 g Cyclohexan, dabei wird gegebenenfalls durch Kühlung die Temperatur bei 10 bis 15°C gehalten. Die viskose Mischung wird dann 14 h bei ca. 15°C nachgerührt.

Danach fügt man 600 g Wasser bei Raumtemperatur zu und destilliert 30 ml eines Azeotrops aus Blausäure, Cyclohexan und Wasser ab, wonach das Reaktionsgemisch praktisch frei von Blausäure ist.

Man fügt weitere 500 ml Cyclohexan zu, trennt die wäßrige Phase ab und wäscht die organische Phase noch 3 mal mit je 500 ml Wasser.

Nach Entfernen des Cyclohexans im Vakuum bleibt das Produkt als nahezu farbloses, viskoses Öl zurück, dessen Jodzahl noch 3,8 beträgt. Der Stickstoffgehalt liegt bei 1,2 %, die Ausbeute beträgt demnach ca. 85 bis 90 %.

### 2. Prüfung der Ventilreinhaltung im Motortest

| Ablagerung [mg]* Ventil-Nr. | | | | |
|---|---|---|---|---|
| | 1 | 2 | 3 | 4 |
| Grundwert | 667 | 521 | 368 | 846 |
| Umsetzungsprodukt aus Beispiel 1 (800 ppm) | 13 | 8 | 0 | 11 |

| | | | | |
|---|---|---|---|---|
| * nach CEC-F-02-T-79 | | | | |

## Patentansprüche

1. Kraftstoffe oder Schmierstoffe, enthaltend geringe Mengen an Verbindungen der Formeln Ia und/oder Ib 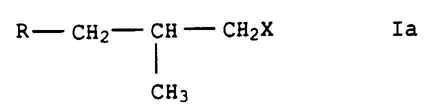 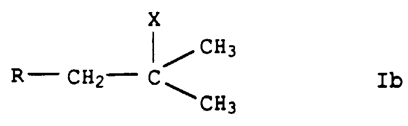 in der
R einen aliphatischen, Alkyl-Seitenketten aufweisenden Kohlenwasserstoffrest mit einem Molekulargewicht (Zahlenmittel) von 250 bis 5000 und
X die Gruppe 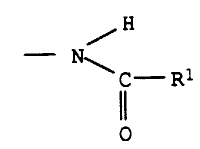 bedeutet, wobei der Rest R¹ Wasserstoff, C₁-C₆-Alkyl, Phenyl oder Alkyl-Phenyl mit 7 bis 14 C-Atomen bedeutet.

2. Kraftstoffe oder Schmierstoffe nach Anspruch 1, dadurch gekennzeichnet, daß R ein von Isobuten und 0 bis 30 Gew.-% n-Buten abgeleiteter Polybutyl- oder Polyisobutylrest ist.

3. Kraftstoffe und Schmierstoffe gemäß Anspruch 1, dadurch gekennzeichnet, daß ein R¹ Wasserstoff bedeutet.

4. Kraftstoffe gemäß Anspruch 1, dadurch gekennzeichnet, daß sie 50 bis 5000 ppm der Verbindungen der Formel Ia und/oder Ib enthalten.

5. Schmierstoffe nach Anspruch 1, dadurch gekennzeichnet, daß sie 0,5 bis 10 Gew.-% der Verbindungen der Formel Ia und/oder Ib enthalten.

6. Kraftstoffe gemäß Anspruch 1, dadurch gekennzeichnet, daß sie neben den Verbindungen der Formel Ia und/oder Ib Kraftstoff-Detergenzien der Formel 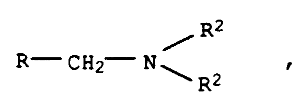 enthalten, in der R die in Anspruch 2 angegebene Bedeutung hat und R² Wasserstoff oder C₁-C₁₀-Alkyl oder C₁-C₈-Aminoalkyl, das noch durch weitere, Aminogruppen tragende C₁-C₆-Alkylreste substituiert sein kann, bedeutet.

## Claims

1. A fuel or lubricant containing small amounts of compounds of the formulae Ia and/or Ib 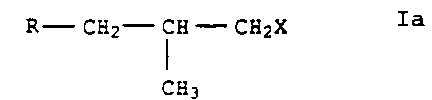 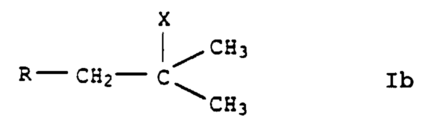 where R is an aliphatic hydrocarbon radical containing alkyl side chains and having a number average molecular weight of from 250 to 5,000, X is 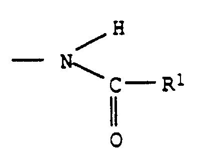 and R¹ is hydrogen, C₁-C₆-alkyl, phenyl or alkylphenyl of 7 to 14 carbon atoms.

2. A fuel or lubricant as claimed in claim 1, wherein R is a polybutyl or polyisobutyl radical derived from isobutene and from 0 to 30% by weight of n-butene.

3. A fuel or lubricant as claimed in claim 1, wherein R¹ is hydrogen.

4. A fuel as claimed in claim 1, which contains from 50 to 5,000 ppm of a compound of the formula Ia and/or Ib.

5. A lubricant as claimed in claim 1, which contains from 0.5 to 10% by weight of a compound of the formula Ia and/or Ib.

6. A fuel as claimed in claim 1, which contains, in addition to a compound of the formula Ia and/or Ib, a fuel detergent of the formula 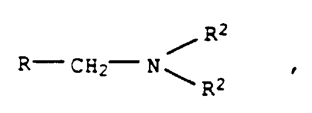 where R has the meanings stated in claim 2 and R² is hydrogen or C₁-C₁₀-alkyl or C₁-C₈-aminoalkyl which may be substituted by further amino-carrying C₁-C₆-alkyl radicals.

## Revendications

1. Carburants ou lubrifiants, contenant de faibles proportions de composés des formules Ia et/ou Ib 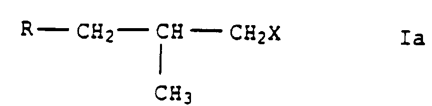 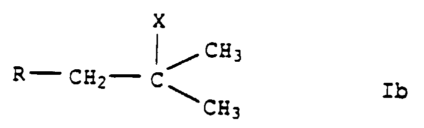 dans lesquelles
R représente un reste d'hydrocarbure aliphatique, présentant des chaînes latérales du type alkyle, d'un poids moléculaire (moyenne en nombre) de 250 à 5000 et
X représente le radical 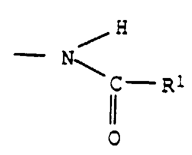 dans lequel le symbole R¹ représente un atome d'hydrogène, un radical alkyle en C₁ à C₆, un radical phényle ou un radical alkylphényle qui comportent de 7 à 14 atomes de carbone.

2. Carburants ou lubrifiants suivant la revendication 1, caractérisés en ce que R représente un radical polybutyle ou polyisobutyle qui dérive de l'isobutène et de 0 à 30% en poids de n-butène.

3. Carburants et lubrifiants suivant la revendication 1, caractérisés en ce que R¹ représente un atome d'hydrogène.

4. Carburants suivant la revendication 1, caractérisés en ce qu'ils contiennent de 50 à 5000 ppm des composés des formules Ia et/ou Ib.

5. Lubrifiants suivant la revendication 1, caractérisés en ce qu'ils contiennent de 0,5 à 10% en poids des composés des formules Ia et/ou Ib.

6. Carburants suivant la revendication 1, caractérisés en ce que, outre les composés des formules Ia et/ ou Ib, ils contiennent des détergents pour carburants répondant à la formule suivante : 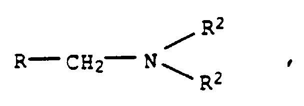 dans laquelle R possède les significations qui lui ont été attribuées dans la revendication 2 et R² représente un atome d'hydrogène ou un radical alkyle en C₁ à C₁₀ ou un radical aminoalkyle en C₁ à C₈, qui peut encore être substitué par d'autres radicaux alkyle en C₁ à C₆ porteurs de groupes amino.
